# EUROPEAN PATENT APPLICATION

(11) **EP 3 689 369 A1**
(43) Date of publication of application: **05.08.2020**
(21) Application number: 19206477.2
(22) Date of filing: 25.02.2014
(51) Int. Cl.: A61K 39/00, A61K 39/395, C07K 16/24, A61P 17/06

(54) **METHODS FOR TREATING PSORIASIS USING AN ANTI-IL-23 ANTIBODY**

(30) Priority: 15.03.2013 US 201361789777 P
(62) Divisional of application: 14711352.6
(71) Applicant: Amgen, Inc, Thousand Oaks, CA 91320-1799 (US)
(72) Inventor: GIBBS, John, P., Mercer Island, WA 98040 (US); TSUJI, Wayne, Seattle, WA 98122 (US); PAN, Wei-Jian, Pullman, WA 99163-5263 (US)
(74) Representative: Dörries, Hans Ulrich

(57) **Abstract**

The invention relates to products and methods for treating psoriasis. The products relate to antibodies that inhibit native human IL-23 while sparing IL-12. One example describes a Phase 1, randomized, double- blind, placebo-controlled, ascending single dose study to evaluate the safety, tolerability, pharmacokinetics and pharmacodynamics of an anti-IL-23 antibody (AMG 139) in healthy subjects and subjects with moderate to severe psoriasis.

SEQUENCE LISTING

<110>  AMGEN INC.
       GIBBS, et al.

<120>  METHODS FOR TREATING PSORIASIS USING AN ANTI-IL-23 ANTIBODY

<130>  32053/47590 PC

<150>  US-61/789,777
<151>  2013-03-15

<160>  2

<170>  PatentIn version 3.5

<210>  1
<211>  124
<212>  PRT
<213>  Homo sapiens

<400>  1

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Glu Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Tyr Thr Ser Ser Trp Tyr Pro Asp Ala Phe Asp
            100                 105                 110

Ile Trp Gly Gln Gly Thr Met Val Thr Val Ser Ser
        115                 120

<210>  2
<211>  111
<212>  PRT
<213>  Homo sapiens

<400>  2

Gln Ser Val Leu Thr Gln Pro Pro Ser Val Ser Gly Ala Pro Gly Gln

1               5                   10                  15

Arg Val Thr Ile Ser Cys Thr Gly Ser Ser Ser Asn Thr Gly Ala Gly
            20                  25                  30

Tyr Asp Val His Trp Tyr Gln Gln Val Pro Gly Thr Ala Pro Lys Leu
        35                  40                  45

Leu Ile Tyr Gly Ser Gly Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Ser
                85                  90                  95

Leu Ser Gly Trp Val Phe Gly Gly Gly Thr Arg Leu Thr Val Leu
            100                 105                 110

## Description

### Field of the Invention

The invention relates to products and methods for treating psoriasis. The products relate to antibodies that inhibit native human IL-23 while sparing IL-12.

### Background

Psoriasis is a common chronic idiopathic inflammatory disease of skin. It affects 1% to 2% of Caucasians including -25 million people in North America and Europe. Both genetic and environmental factors play key roles in the pathogenesis of psoriasis, which is noted histopathologically by marked thickening of the epidermis, alterations in keratinocyte proliferation and differentiation, and a genetic program similar to that observed in wound repair. The trigger for this altered keratinocyte response is thought to be activation of the cellular immune system, and numerous studies have implicated T-cells, dendritic cells and various inflammatory cytokines and chemokines in disease pathogenesis (Nestle FO, Kaplan DH, et al. Psoriasis. N Engl J Med. 2009;361(5):496-509; Griffiths CE and Barker JN. Lancet. 2007;370(9583):263-271; Lowes MA, Bowcock AM, et al. Nature. 2007;445(7130):866-873; Nickoloff BJ and Nestle FO. J Clin Invest. 2004; 113(12):1664-1675.

Interleukin 23 (IL-23), expression is increased in psoriatic lesional tissue. IL-23 is a heterodimeric cytokine and a potent inducer of pro-inflammatory cytokines. IL-23 is related to the heterodimeric cytokine Interleukin 12 (IL-12) both sharing a common p40 subunit. In IL-23, a unique p19 subunit is covalently bound to the p40 subunit. In IL-12, the unique subunit is p35 (Oppmann et al., Immunity, 2000, 13: 713-715). Like IL-23 is expressed by antigen presenting cells (such as dendritic cells and macrophages) in response to activation stimuli such as CD40 ligation, Toll-like receptor agonists and pathogens. IL-23 binds a heterodimeric receptor comprising an IL-12Rβ1 subunit (which is shared with the IL-12 receptor) and a unique receptor subunit, IL-23R.

IL-23 acts on activated and memory T cells and promotes survival and expansion of the T cell subset, Th17. Th17 cells produce proinflammatory cytokines including IL-6, IL-17, TNFα, IL-22 and GM-CSF. IL-23 also acts on natural killer cells, dendritic cells and macrophages to induce pro-inflammatory cytokine expression. Unlike IL-23, IL-12 induces the differentiation of naive CD4+ T cells into mature Th1 IFNγ-producing effector cells, and induces NK and cytotoxic T cell function by stimulating IFNγ production. Th1 cells driven by IL-12 were previously thought to be the pathogenic T cell subset in many autoimmune diseases, however, more recent animal studies in models of inflammatory bowel disease, psoriasis, inflammatory arthritis and multiple sclerosis, in which the individual contributions of IL-12 versus IL-23 were evaluated have firmly established that IL-23, not IL-12, is the key driver in autoimmune/inflammatory disease (Ahern et al., Immun. Rev. 2008 226:147-159; Cua et al., Nature 2003 421:744-748; Yago et al., Arthritis Res and Ther. 2007 9(5): R96). It is believed that IL-12 plays a critical role in the development of protective innate and adaptive immune responses to many intracellular pathogens and viruses and in tumor immune surveillance. See Kastelein, et al., Annual Review of Immunology, 2007, 25: 221-42; Liu, et al., Rheumatology, 2007, 46(8): 1266-73; Bowman et al., Current Opinion in Infectious Diseases, 2006 19:245-52; Fieschi and Casanova, Eur. J. Immunol. 2003 33:1461-4; Meeran et al., Mol. Cancer Ther. 2006 5: 825-32; Langowski et al., Nature 2006 442: 461-5. As such, IL-23 specific inhibition (sparing IL-12 or the shared p40 subunit) should have a potentially superior safety profile compared to dual inhibition of IL-12 and IL-23.

IL-23p19 and IL-12/23p40 mRNA are increased in psoriatic lesional skin as compared to non-lesional skin; 22- and 12-fold mean increase, respectively. The expression of IL-12p35 mRNA did not differ significantly between paired lesional and nonlesional skin (Lee E, Trepicchio WL, et al. J Exp Med. 2004; 199(1):125-130.). These data suggest IL-23 is upregulated in psoriatic lesional tissue while IL-12 is not. IL-23 protein has been demonstrated to be upregulated in psoriatic lesional skin as well through immunohistochemical analysis. Anti-IL-23p19 antibody staining showed increased expression in both the epidermis and the dermis in lesional psoriatic skin as compared to normal (and nonlesional) skin (Piskin G, Sylva-Steenland RM, et al. In vitro and in situ expression of IL-23 by keratinocytes in healthy skin and psoriasis lesions: enhanced expression in psoriatic skin. J Immunol. 2006;176(3):1908-1915). IL-23 levels decrease with clinical improvement of PsO following effective treatment of disease (either UV or anti-TNF treatment) providing a direct correlation between overproduction of IL-23 and active psoriasis (Fitch E, Harper E, et al. Pathophysiology of psoriasis: recent advances on IL-23 and Th17 cytokines. Curr Rheumatol Rep. 2007;9(6):461-4).

A genome-wide association study was conducted in psoriasis patients using a collection of > 25,000 primarily functional SNPs in 3 independent case-control sample sets. In this study they found a highly significant association with a SNP in the 3'UTR of IL-12/23p40. Multiple SNPs in the IL-12 (p35) and IL-23 (p19) ligand and receptor chains (IL-12Rβ1, IL-12Rβ2, and IL-23R) were individually genotyped. Two SNPs in the IL-23R were highly associated with psoriasis while there was no association with the other ligand and receptor chains (Cargill M, Schrodi SJ, et al. Am J Hum Genet. 2007;80(2):273-290). The finding that common variants in both IL-12/23p40 and IL-23R are associated with psoriasis risk provides genetic evidence that the IL-23 pathway plays an important role in psoriasis pathogenesis.

Current approved therapies for psoriasis include topical agents (eg, corticosteroids, coal tar preparations, retinoids, phototherapy); systemic therapies (eg, methotrexate, retinoids, cyclosporin); and biologics (eg, etanercept, adalimumab, alefacept, ustekinumab). Despite these available therapies many patients remain untreated, do not respond to therapy, or suffer from toxicities associated with systemic or phototherapy, with significant skin involvement and disability.

It is contemplated herein that there is a need for new modalities for the treatment of psoriasis that specifically target IL-23 without the potential risks associated with inhibition of IL-12. Provided herein are methods for the treatment of psoriasis using fully human therapeutic agents that are able to inhibit native human IL-23 while sparing IL-12.

### Summary

Provided herein are methods of treating psoriasis in a subject in need thereof comprising administering to the subject an anti-IL-23 antibody in an amount and at an interval of: 15 - 54 mg every 0.5 - 1.5 months; 55 - 149 mg every 1.5 - 4.5 months; 150 - 299 mg every 4 - 8 months; or 300 - 1100 mg every 4 - 12 months. In some embodiments, the amount and interval are: 15 - 21 mg every 0.5 - 1.0 month; 55 - 70 mg every 1.5 - 3.0months; 150 - 260 mg every 4 - 6 months; or 300 - 700 mg every 4 - 8 months. In some embodiments, the amount and interval are: 21 mg every month; 70 mg every 3 months; 210mg every 6 months; or 700 mg every 6 months. In some embodiments, the amount and interval are: 210 mg every 3 months or 700 mg every 3 months. In some embodiments, the amount and interval are: 210 mg every 1 month or 700 mg every 1 month. In some embodiments of the methods, the anti-IL23 antibody is administered IV. In some embodiments of the methods, the anti-IL23 antibody is administered SC. In some embodiments of the methods, the anti-IL-23 antibody is AMG 139.

Also provided herein are methods of treating psoriasis in a subject in need thereof comprising administering to the subject an amount of an anti-IL-23 antibody in an amount and at an interval sufficient to achieve and/or maintain a quantity of anti-IL-23 antibody per volume of serum of between 12.5 ng/ml and 1000 ng/ml. In some embodiments, the quantity of an anti-IL-23 antibody per volume of serum is at least 10 ng/ml. In some embodiments, the quantity of an anti-IL-23 antibody per volume of serum is selected from the group consisting of: at least 25 ng/ml; at least 50 ng/ml; at least 60 ng/ml; at least 70 ng/ml; at least 75 ng/ml; and at least 80 ng/ml. In some embodiments, the quantity of an anti-IL-23 antibody per volume of serum is between 85 ng/ml and 100 ng/ml. In some embodiments, the quantity of an anti-IL-23 antibody per volume of serum is between 70 ng/ml and 150 ng/ml. In some embodiments the quantity of an anti-IL-23 antibody per volume of serum is is between 50 ng/ml and 250 ng/ml. In some embodiments, the quantity of an anti-IL-23 antibody per volume of serum is is between 40 ng/ml and 500 ng/ml. In some embodiments, the quantity of an anti-IL-23 antibody per volume of serum is between 25 ng/ml and 750 ng/ml. In some embodiments, the quantity of an anti-IL-23 antibody per volume of serum is between 10 ng/ml and 1,000 ng/ml. In some embodiments of the methods, the anti-IL23 antibody is administered IV. In some embodiments of the methods, the anti-IL23 antibody is administered SC. In some embodiments of the methods, the anti-IL-23 antibody is AMG 139.

### Brief Description of the Drawings

Figure 1 presents the results of the pharmacokinetic analysis of an ascending single dose study of subcutaneous administration of AMG 139 in healthy subjects (HS). The results shown illustrate the mean (± SD) serum AMG 139 concentration-time profiles.
Figure 2 presents the results of the pharmacokinetic analysis of an ascending single dose study of intravenous administration of AMG 139 in healthy subjects (HS). The results shown illustrate the mean (± SD) serum AMG 139 concentration-time profiles.
Figure 3 presents the results of the pharmacokinetic analysis of an ascending single dose study of subcutaneous AMG 139 administration in psoriasis subjects (PsO). The results shown illustrate the mean (± SD) serum AMG 139 concentration-time profiles.
Figure 4 presents the results of the pharmacokinetic analysis of an ascending single dose study of intravenous AMG 139 administration in psoriasis subjects (PsO). The results shown illustrate the mean (± SD) serum AMG 139 concentration-time profiles.
Figure 5 presents the results of Psoriasis Area and Severity Index (PASI) score assessment in PsO subjects from the single ascending dose study. The results shown illustrate the mean PASI score (± SD) at time points throughout the study.
Figure 6 presents the results of Psoriasis Area and Severity Index (PASI) score assessment (normalized to baseline) in PsO subjects from the single ascending dose study. The results shown illustrate the mean improvement of PASI score from baseline (± SD) at time points throughout the study.
Figure 7 presents the pharmacokinetic structural model used in developing the AMG 139 quantitative population PK model based on data from Example 1.
Figure 8 presents the results of a diagnostic visual predictive check of the AMG 139 population PK model. The results shown illustrate the mean (solid line) and 90% confidence interval (dashed line) AMG 139 concentration-time profile after simulating 1000 clinical trials. Each point represents actual, observed concentrations from subjects.
Figure 9 presents the results of multiple diagnostic visual predictive checks of the AMG 139 population PK model. The results illustrate correlations between observed AMG 139 concentrations and that of population and individual predicted concentrations, as well as the weighted residuals of model fitting between population predicted concentrations and time.
Figure 10 presents the results of a correlation analysis between body weight and PK parameters. The results illustrate a positive correlation in individual CL and V with body weight for the combined population of healthy and PsO subjects.

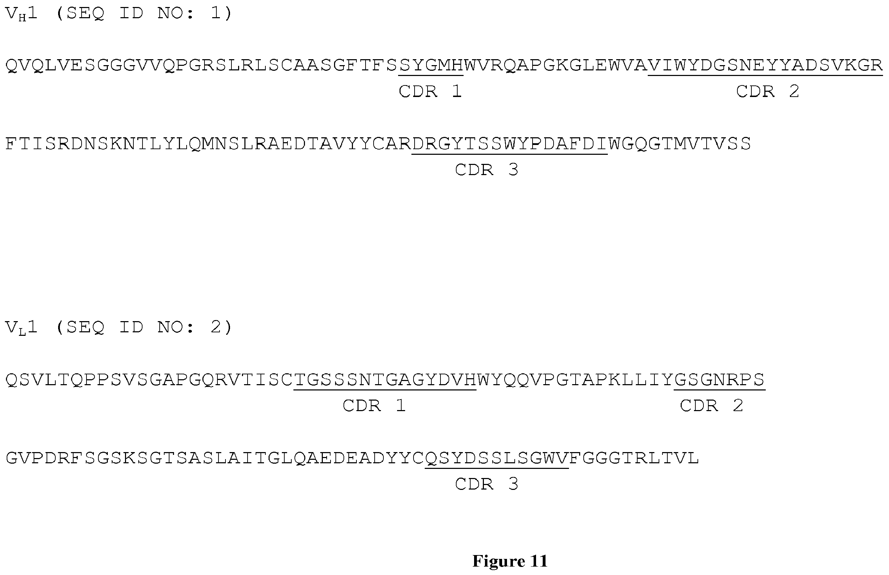

Figure 11 presents the amino acid sequences of AMG 139 heavy and light chain variable regions.

### Detailed Description

Provided herein are methods for treating psoriasis in a subject in need thereof comprising administering to the subject an amount of a human monoclonal antibody that specifically binds IL-23. In some embodiments, the anti-IL-23 antibody specifically binds IL-23 but spares IL-12.

The terms "treating", and "treatment" and the like are used herein to generally mean obtaining a desired pharmacological, physiological or therapeutic effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease and/or its symptoms or conditions. The invention is directed towards treating a patient's suffering from disease related to pathological inflammation. The present invention is involved in preventing, inhibiting, or relieving adverse effects attributed to pathological inflammation over long periods of time and/or are such caused by the physiological responses to inappropriate inflammation present in a biological system over long periods of time.

In one aspect, the present invention provides methods of treating a subject. The method can, for example, have a generally salubrious effect on the subject, e.g., it can increase the subject's expected longevity. Alternatively, the method can, for example, treat, prevent, cure, relieve, or ameliorate ("treat") a disease, disorder, condition, or illness ("a condition"). In one embodiment, the present invention provides a method of treating a condition in a subject comprising administering the pharmaceutical composition comprising an specific antibody to the subject, wherein the condition is treatable by reducing the activity (partially or fully) of IL-23 in the subject. Treating encompasses both therapeutic administration (i.e., administration when signs and symptoms of the disease or condition are apparent) as well prophylactic or maintenance therapy (i.e., administration when the disease or condition is quiescent), as well as treating to induce remission and/or maintain remission. Accordingly, the severity of the disease or condition can be reduced (partially, significantly or completely), or the signs and symptoms can be prevented or delayed (delayed onset, prolonged remission, or quiescence).

Among the conditions to be treated in accordance with the present invention are conditions in which IL-23 is associated with or plays a role in contributing to the underlying disease or disorder or otherwise contributes to a negative symptom. Such conditions include skin disorders such as psoriasis, plaque psoriasis, guttate psoriasis, inverse psoriasis, pustular psoriasis, erythrodermic psoriasis, dermatitis and atopic dermatitis.

The term "efficacy" as used herein in the context of a dosage regimen refers to the effectiveness of a particular treatment regimen. Efficacy can be measured based on change the course of the disease in response to an agent of the present invention. In one embodiment, an antigen binding protein (for example, an anti-IL-23 antibody) is administered to the subject in an amount and for a time sufficient to induce an improvement, preferably a sustained improvement, in at least one indicator that reflects the severity of the disorder that is being treated. Various indicators that reflect the extent of the subject's illness, disease or condition may be assessed for determining whether the amount and time of the treatment is sufficient. Such indicators include, for example, clinically recognized indicators of disease severity, symptoms, or manifestations of the disorder in question.

In one embodiment, an improvement is considered to be sustained if the subject exhibits the improvement on at least two occasions separated by two to four weeks. In another embodiment, an improvement is considered to be sustained if the subject exhibits the improvement on at least two occasions separated by two to four months; in a further embodiment, an improvement is considered to be sustained if the subject exhibits the improvement on at least two occasions separated by six to twelve months. The degree of improvement generally is determined by a physician, who may make this determination based on signs, symptoms, biopsies, or other test results, and who may also employ questionnaires that are administered to the subject, such as quality-of-life questionnaires developed for a given disease.

The IL-23 specific antibody may be administered to achieve an improvement in a subject's condition. Improvement may be indicated by a decrease in an index of disease activity, by amelioration of clinical symptoms or by any other measure of disease activity. On such index of disease is the psoriasis area and severity index (PASI). PASI is a measurement of of the average redness, thickness, and scaliness of the lesions, each graded on a scale of 0-4, weighed by the area of involvement. Psoriasis Target Lesion Assessment Score, is an index for assessing the severity of individual skin lesions. The score is based on the sum of the evaluation of plaque elevation, amount and degree of scaling or degree of erythema, and target lesion response to treatment. Another disease index is the National Psoriasis Foundation Psoriasis Score (NSF-PS). The degree of improvement generally is determined by a physician, who may make this determination based on signs, symptoms, (such as a phychian global assessment (PGA)), an overall lision assessment (OLA), biopsies, whole body photographs, or other test results, and who may also employ questionnaires that are administered to the subject, such as quality-of-life questionnaires developed for a given disease.

In one embodiment, an improvement is considered to be sustained if the subject exhibits the improvement on at least two occasions separated by two to four weeks. In another embodiment, an improvement is considered to be sustained if the subject exhibits the improvement on at least two occasions separated by two to four months; in a further embodiment, an improvement is considered to be sustained if the subject exhibits the improvement on at least two occasions separated by six to twelve months. In another embodiment, improvement is considered to be achieved when the subject exhibits at least 50, 55, 60, 65, 70, 75, 80, 85, 90, 95, or 100% improvement in PASI score.

Treatment of a subject with an IL-23 specific antibody may be given in an amount and/or at sufficient interval to achieve and/or maintain a certain quantity of IL-23-specific antibody per volume of serum, using, for example, an assay as described herein. For example, the heterodimer specific antibody is given to achieve from 12.5 ng/ml to 1000ng/ml. In one embodiment, the heterodimer specific antibody is given to achieve at least 12.5 ng/ml, 25 ng/ml, 50 ng/ml, 60 ng/ml, 70 ng/ml, 75 ng/ml, 80 ng/ml, 85 ng/ml, 90 ng/ml, 95 ng/ml, 100 ng/ml, 150 ng/ml, 200 ng/ml, 500 ng/ml, or 990 ng/ml. Those of skill in the art will understand that the amounts given here apply to a full-length antibody or immunoglobulin molecule; if an antigen binding fragment thereof is used, the absolute quantity will differ from that given in a manner that can be calculated based on the molecular weight of the fragment.

Treatment of a subject with an IL-23 specific antibody may be given in an amount and at an interval of 15 - 54 mg every 0.5 - 1.5 months; 55 - 149 mg every 1.5 - 4.5 months; 150 - 299 mg every 4 - 8 months; or 300 - 1100 mg every 14 - 8 months. In one embodiment the amount and interval are selected from the group consisting of: 21 mg every month; 70 mg every 3 months; 210 mg every 6 months; or 700 mg every 6 months.

Both ubcutaneous and intravenous administration of AMG139 significantly reduced the symptoms of psoriasis as measured by the PASI scoring system. In some embodiments, administration of AMG139 at the dosages and administration schedules described above may be used to reduce the PASI score in a patient having psoriasis by at least 10, 15, 20, 25, 30, 35, 40, 45, 50, 55, 60, 65, 70, 75, 80, 85, 90, 95, or 100%.

It is understood that the methods of treating the diseases described herein would administer an effective amount of an anti-IL-23 antibody. Depending on the indication to be treated, a therapeutically effective amount is sufficient to cause a reduction in at least one symptom of the targeted pathological condition by at least about 5%, 10%, 15%, 20%, 25%, 30%, 35%, 40%, 45%, 50%, 55%, 60%, 65%, 70%, 75%, 80%, 85%, 90%, 95%, or more, relative to untreated subjects.

Administration and dosage regimens of an anti-IL-23 antibody can be adjusted to provide an effective amount for an optimum therapeutic response. For example, a single bolus can be administered, several divided doses can be administered over time or the dose can be proportionally reduced or increased as indicated by the exigencies of the therapeutic situation. The anti-IL-23 antibody may be administered by any suitable technique, including but not limited to, parenterally, topically, or by inhalation. If injected, the pharmaceutical composition can be administered, for example, via intra-articular, intravenous, intramuscular, intralesional, intraperitoneal or cutaneous routes (including intra-, trans- or sub- dermal, and subcutaneous), by bolus injection, or continuous infusion. In some embodiments, the pharmaceutical composition is administered by an intravenous route. In some embodiments the pharmaceutical composition is administered by a subcutaneous route. In further embodiments, the compositions are administered by oral, buccal, rectal, intratracheal, gastric, or intracranial routes. Localized administration, e.g. at a site of disease or injury is contemplated, for example, by enema or suppository for conditions involving the gastrointestinal tract. Also contemplated are transdermal delivery and sustained release from implants. Delivery by inhalation includes, for example, nasal or oral inhalation, use of a nebulizer, inhalation of the antagonist in aerosol form, and the like. Other alternatives include eyedrops; oral preparations including pills, syrups, lozenges or chewing gum; and topical preparations such as lotions, gels, sprays, and ointments.

Advantageously, IL-23 antibodies are administered in the form of a composition comprising one or more additional components such as a physiologically acceptable carrier, excipient or diluent. Optionally, the composition additionally comprises one or more physiologically active agents for combination therapy. A pharmaceutical composition may comprise an anti-IL-23 antibody together with one or more substances selected from the group consisting of a buffer, an antioxidant such as ascorbic acid, a low molecular weight polypeptide (such as those having fewer than 10 amino acids), a protein, an amino acid, a carbohydrate such as glucose, sucrose or dextrins, a chelating agent such as EDTA, glutathione, a stabilizer, and an excipient. Neutral buffered saline or saline mixed with conspecific serum albumin are examples of appropriate diluents. In accordance with appropriate industry standards, preservatives such as benzyl alcohol may also be added. The composition may be formulated as a lyophilizate using appropriate excipient solutions (e.g., sucrose) as diluents. The anti-IL-23 antibody can be provided at a concentration of 50 to 200 mg/ml. Exemplary formulations useful for the present invention are those that include a glutamic acid, citric acid or acetic acid buffer as an appropriate pH, from 4.5 to 5.2, an excipient such as sucrose, glycine, proline, glycerol, and/or sorbitol at an appropriate concentration such as 1 to 20% (w/v), and a surfactant such as a non-ionic surfactant like polysorbate (polysorbate 20 or 80) or poloxamers (poloxamer 1888) at an appropriate concentration of 0.001% - 0.1% (w/v). Such formulations are disclosed in US Patent No. 6171586 and WIPO Published Applications Nos: WO20100027766 and WO2011088120. In some embodiments, the formulations comprise sodium acetate, sucrose and polysorbate 20. In some embodiments, the formulations comprise 70 mg/mL AMG 139, 10 mM sodium acetate, 9% (w/v) sucrose and 0.004% (w/v) polysorbate 20, at pH 5.2. Suitable components are nontoxic to recipients at the dosages and concentrations employed. Further examples of components that may be employed in pharmaceutical formulations are presented in any Remington's Pharmaceutical Sciences including the 21st Ed. (2005), Mack Publishing Company, Easton, PA.

Kits for use by medical practitioners include an anti-IL-23 antibody and a label or other instructions for use in treating any of the conditions discussed herein. In one embodiment, the kit includes a sterile preparation of one or more IL-23 binding antigen binding proteins, which may be in the form of a composition as disclosed above, and may be in one or more vials.

Particular embodiments of methods of the invention involve the use of an anti-IL-23 antibody and one or more additional IL-23 antagonists, as described in US Patents US 7,491,391; US 7,807,414; US7,872,102; US7,807,160; US8362212; US7,935,344; US 7,790,862; US2012282269; US Published Patent Applications US 2009-0123479; US 20120128689; and US2012264917 and WIPO Publications WO1999/05280, WO2007/0244846, WO2007/027714, WO2007/076524, WO2007/147019, WO2008/103473, WO2008/103432, WO2009/043933, WO2009/082624 WO 12/009760.

Also provided are IL-23 antibodies administered alone or in combination with other agents useful for treating the condition with which the patient is afflicted. Topical medications (e.g., steroids, coal tar, anthralin, Dead Sea salts, various natural oils, vitamin D3 and its analogs, sunshine, topical retinoids), phototherapy (e.g., ultraviolet light, photochemotherapy (PUVA)), and internal medications (e.g., methotrexate, systemic steroids, oral retinoids, cyclosporine,). When multiple therapeutics are co-administered, dosages may be adjusted accordingly, as is recognized or known in the pertinent art.

In every case where a combination of molecules and/or other treatments is used, the individual molecule(s) and/or treatment(s) can be administered in any order, over any length of time, which is effective, e.g., simultaneously, consecutively, or alternately. In one embodiment, the method of treatment comprises completing a first course of treatment with one molecule or other treatment before beginning a second course of treatment. The length of time between the end of the first course of treatment and beginning of the second course of treatment can be any length of time that allows the total course of therapy to be effective, e.g., seconds, minutes, hours, days, weeks, months, or even years.

The terms "polypeptide" or "protein" means a macromolecule having the amino acid sequence of a native protein, that is, a protein produced by a naturally-occurring and non-recombinant cell; or it is produced by a genetically-engineered or recombinant cell, and comprise molecules having the amino acid sequence of the native protein, or molecules having one or more deletions from, insertions to, and/or substitutions of the amino acid residues of the native sequence. The term also includes amino acid polymers in which one or more amino acids are chemical analogs of a corresponding naturally-occurring amino acid and polymers. The terms "polypeptide" and "protein" encompass IL-23 antibodies and sequences that have one or more deletions from, additions to, and/or substitutions of the amino acid residues of the antigen binding protein sequence. The term "polypeptide fragment" refers to a polypeptide that has an amino-terminal deletion, a carboxyl-terminal deletion, and/or an internal deletion as compared with the full-length native protein. Such fragments may also contain modified amino acids as compared with the native protein. In certain embodiments, fragments are about five to 500 amino acids long. For example, fragments may be at least 5, 6, 7, 8, 9, 10, 11, 12, 13, 14, 15, 16, 17, 18, 19, 20, 50, 70, 100, 110, 150, 200, 250, 300, 350, 400, or 450 amino acids long. Useful polypeptide fragments include immunologically functional fragments of antibodies, including binding domains. In the case of an anti-IL-23 antibody, useful fragments include but are not limited to one or more CDR regions, a variable domain of a heavy or light chain, a portion of an antibody chain, a portion of a variable region including less than three CDRs, and the like.

The term "isolated protein" refers to a protein, such as an antigen binding protein (an example of which could be an antibody), that is purified from proteins or polypeptides or other contaminants that would interfere with its therapeutic, diagnostic, prophylactic, research or other use. As used herein, "substantially pure" means that the described species of molecule is the predominant species present, that is, on a molar basis it is more abundant than any other individual species in the same mixture. In certain embodiments, a substantially pure molecule is a composition wherein the object species comprises at least 50% (on a molar basis) of all macromolecular species present. In other embodiments, a substantially pure composition will comprise at least 80%, 85%, 90%, 95%, or 99% of all macromolecular species present in the composition. In certain embodiments, an essentially homogeneous substance has been purified to such a degree that contaminating species cannot be detected in the composition by conventional detection methods and thus the composition consists of a single detectable macromolecular species.

A "variant" of a polypeptide (e.g., an antigen binding protein such as an antibody) comprises an amino acid sequence wherein one or more amino acid residues are inserted into, deleted from and/or substituted into the amino acid sequence relative to another polypeptide sequence. Variants include fusion proteins. A "derivative" of a polypeptide is a polypeptide that has been chemically modified in some manner distinct from insertion, deletion, or substitution variants, e.g., via conjugation to another chemical moiety.

The terms "naturally occurring" or "native" as used throughout the specification in connection with biological materials such as polypeptides, nucleic acids, host cells, and the like, refers to materials which are found in nature, such as native human IL-23. In certain aspects, recombinant antigen binding proteins that bind native IL-23 are provided. In this context, a "recombinant protein" is a protein made using recombinant techniques, i.e., through the expression of a recombinant nucleic acid as described herein. Methods and techniques for the production of recombinant proteins are well known in the art.

The term "antibody" refers to an intact immunoglobulin of any isotype, or a fragment thereof that can compete with the intact antibody for specific binding to the target antigen, and includes, for instance, chimeric, humanized, fully human, and bispecific antibodies. An antibody as such is a species of an antigen binding protein. Unless otherwise indicated, the term "antibody" includes, in addition to antibodies comprising two full-length heavy chains and two full-length light chains, derivatives, variants, fragments, and muteins thereof, examples of which are described below. An intact antibody generally will comprise at least two full-length heavy chains and two full-length light chains, but in some instances may include fewer chains such as antibodies naturally occurring in camelids which may comprise only heavy chains. Antibodies may be derived solely from a single source, or may be "chimeric," that is, different portions of the antibody may be derived from two different antibodies as described further below. The antigen binding proteins, antibodies, or binding fragments may be produced in hybridomas, by recombinant DNA techniques, or by enzymatic or chemical cleavage of intact antibodies.

The term "functional fragment" (or simply "fragment") of an antibody or immunoglobulin chain (heavy or light chain), as used herein, is an antigen binding protein comprising a portion (regardless of how that portion is obtained or synthesized) of an antibody that lacks at least some of the amino acids present in a full-length chain but which is capable of specifically binding to an antigen. Such fragments are biologically active in that they bind specifically to the target antigen and can compete with other antigen binding proteins, including intact antibodies, for specific binding to a given epitope. In one aspect, such a fragment will retain at least one CDR present in the full-length light or heavy chain, and in some embodiments will comprise a single heavy chain and/or light chain or portion thereof. These biologically active fragments may be produced by recombinant DNA techniques, or may be produced by enzymatic or chemical cleavage of antigen binding proteins, including intact antibodies. Fragments include, but are not limited to, immunologically functional fragments such as Fab, Fab', F(ab')2, Fv, domain antibodies and single-chain antibodies, and may be derived from any mammalian source, including but not limited to human, mouse, rat, camelid or rabbit. It is contemplated further that a functional portion of the antigen binding proteins disclosed herein, for example, one or more CDRs, could be covalently bound to a second protein or to a small molecule to create a therapeutic agent directed to a particular target in the body, possessing bifunctional therapeutic properties, or having a prolonged serum half-life.

An "antigen binding protein" as used herein means a protein that specifically binds a specified target antigen; the antigen as provided herein is IL-23, particularly human IL-23, including native human IL-23. Antigen binding proteins as provided herein interact with at least a portion of the unique p19 subunit of IL-23, detectably binding IL-23; but do not bind with any significance to IL-12 (e.g., the p40 and/or the p35 subunits of IL-12), thus "sparing IL-12". As a consequence, the antigen binding proteins provided herein are capable of impacting IL-23 activity without the potential risks that inhibition of IL-12 or the shared p40 subunit might incur. The antigen binding proteins may impact the ability of IL-23 to interact with its receptor, for example by impacting binding to the receptor, such as by interfering with receptor association. In particular, such antigen binding proteins totally or partially reduce, inhibit, interfere with or modulate one or more biological activities of IL-23. Such inhibition or neutralization disrupts a biological response in the presence of the antigen binding protein compared to the response in the absence of the antigen binding protein and can be determined using assays known in the art and described herein. Antigen binding proteins provided herein inhibit IL-23-induced proinflammatory cytokine production, for example IL-23-induced IL-22 production in whole blood cells and IL-23-induced IFNγ expression in NK and whole blood cells. Reduction of biological activity can be about 20%, 30%, 40%, 50%, 60%, 70%, 80%, 85%, 90%, 91%, 92%, 93%, 94%,95%, 96%, 97% 98%, 99% or more.

Certain antigen binding proteins described herein are antibodies, or are derived from antibodies. Such antigen binding proteins include, but are not limited to, monoclonal antibodies, bispecific antibodies, minibodies, domain antibodies, synthetic antibodies, antibody mimetics, chimeric antibodies, humanized antibodies, human antibodies, antibody fusions, antibody conjugates, single chain antibodies, and fragments thereof, respectively. In some instances, the antigen binding protein is an immunological fragment of an antibody (e.g., a Fab, a Fab', a F(ab')2, or a scFv).

Certain antigen binding proteins that are provided may comprise one or more CDRs as described herein (e.g., 1, 2, 3, 4, 5, 6 or more CDRs). In some instances, the antigen binding protein comprises (a) a polypeptide structure and (b) one or more CDRs that are inserted into and/or joined to the polypeptide structure. The polypeptide structure can take a variety of different forms. For example, it can be, or comprise, the framework of a naturally occurring antibody, or fragment or variant thereof, or may be completely synthetic in nature. Examples of various polypeptide structures are further described below.

An antigen binding protein of the invention is said to "specifically bind" its target antigen when the dissociation equilibrium constant (KD) is ≤ 10⁻⁸ M. The antigen binding protein specifically binds antigen with "high affinity" when the KD is ≤ 5 x 10⁻⁹ M, and with "very high affinity" when the the KD is ≤ 5 x 10⁻¹⁰ M. In one embodiment the antigen binding protein will bind to human IL-23 with a KD of ≤ 5 x 10⁻¹² M, and in yet another embodiment it will bind with a KD ≤ 5 x 10⁻¹³ M. In another embodiment of the invention, the antigen binding protein has a KD of ≤ 5 x 10⁻¹² M and an Koff of about ≤5x10⁻⁶ 1/s. In another embodiment, the Koff is ≤ 5x10⁻⁷1/s.

In embodiments where the antigen binding protein is used for therapeutic applications, an antigen binding protein can reduce, inhibit, interfere with or modulate one or more biological activities of IL-23, such inducing production of proinflammatory cytokines. IL-23 has many distinct biological effects, which can be measured in many different assays in different cell types; examples of such assays and known see for example US Patent Application No: US 2013-0004501, the disclosure of which is incorporated by reference herein Exemplary IL-23 antibodies are disclosed US Patent Application No: US 2013-0004501.

As used herein, "AMG 139" refers to an intact AMG 139 immunoglobulin or to an antigen binding portion thereof that competes with the intact antibody for specific binding, unless otherwise specified. AMG 139 also includes antibodies (or fragments thereof) that are identical or similar to AMG 139 in amino acid sequence, particularly in the variable regions, or in the CDRs thereof (however, variations in the constant regions are also contemplated). For example, a useful AMG 139 polypeptide has an amino acid sequence that is 85%, 90%, 92%, 95%, 98%, 99% or 100% identical to that of an AMG 139 polypeptide disclosed herein. In another embodiment, a useful polypeptide is between 80% and 100% identical to AMG 139.

AMG139 is a human antibody that specifically recognizes the native human IL-23 heterodimer, but does not bind with any significance to the human IL-12 heterodimer. AMG139 inhibits IL-23-induced proinflammatory cytokine production, for example IL-23-induced IL-22 production in whole blood cells and IL-23-induced IFNγ expression in NK and whole blood cells. In some embodiments, AMG 139 is an isolated, IL-23 specific antigen binding protein having a heavy chain variable region comprising CDR1, CDR2 and CDR3 from SEQ ID NO:1, and a light chain variable region comprising CDR1, CDR2 and CDR3 from SEQ ID NO:2. In some embodiments, AMG 139 is an isolated, IL-23 specific antigen binding protein wherein the heavy chain variable region is at least 90% identical to SEQ ID NO:1, and the light chain variable region is at least 90% identical to CDR1, CDR2 and CDR3 from SEQ ID NO:2. See, WO 2011/056600 published May 11, 2011.

Where a range of values is provided, it is understood that each intervening value (to the tenth of the unit of the lower limit unless the context clearly dictates otherwise) between the upper and lower limit of that range, and any other stated or intervening value or smaller range, in that stated range is encompassed within the invention. The upper and lower limits of smaller ranges may independently be included in the smaller range, subject to any specifically excluded limit in the stated range. Where the stated range includes one or both of the limits, ranges excluding either both of those included limits are also included in the invention.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

All patents and other publications identified are expressly incorporated herein by reference in their entirety for the purpose of describing and disclosing, for example, the methodologies described in such publications that might be used in connection with information described herein.

The following examples, both actual and prophetic, are provided for the purpose of illustrating specific embodiments or features of the instant invention and do not limit its scope.

### Example 1

This example describes a Phase 1, randomized, double-blind, placebo-controlled, ascending single dose study to evaluate the safety, tolerability, pharmacokinetics (PK) and pharmacodynamics (PD) of an anti-IL-23 antibody (AMG 139) in healthy subjects (HS) and subjects with moderate to severe psoriasis (PSO); ClinicalTrials.gov Identifier: NCT01094093).

A total of 73 subjects were randomized into the study; 56 healthy adults were randomized in Part A and received AMG 139 as a single SC dose (7, 21, 70, or 210 mg) or single IV dose (210, 420, or 700 mg) or placebo, and 17 subjects with moderate to severe PsO were randomized in Part B and received AMG 139 as a single SC dose (21, 70, or 210 mg) or single IV dose (700 mg) or placebo, see Table 1.

**Table 1. Dosage and Administration Route for Each Cohort**

| Cohorts | Dosage (mg) | Route | Number of Subjects (AMG 139:placebo) |
|---|---|---|---|
| PART A | Healthy Subjects (HS) | | |
| Cohort A1 | 7 | SC | 2 (1:1) |
| | | | 6 (5:1) |
| Cohort A2 | 21 | SC | 8 (6:2) |
| Cohort A3 | 70 | SC | 8 (6:2) |
| Cohort A4 | 210 | SC | 8 (6:2) |
| Cohort A5 | 210 | IV | 8 (6:2) |
| Cohort A6 | 420 | IV | 8 (6:2) |
| Cohort A7 | 700 | IV | 8 (6:2) |

| PART B | Subjects with PsO | | |
|---|---|---|---|
| Cohort B1 | 21 | SC | 4 (3:1) |
| Cohort B2 | 70 | SC | 4 (3:1) |
| Cohort B3 | 210 | SC | 4 (3:1) |
| Cohort B4 | 700 | IV | 4 (3:1) |

Serial blood samples were taken at scheduled time points in Part A to day 85 for cohorts A1, A2 and A3 and day 169 for cohorts A5, A6 and A7. For Part B, scheduled time points were to day 113 for cohorts B1 and B2 (21 and 70 mg AMG139 SC, respectfully). Day 169 for cohorts B3 and B4 (210 SC and 700IV AMG139, respectfully).

To measure the amount of AMG 139 in serum from a subject, capture antibody (mouse anti-AMG 139 1F2 mAb) was passively adsorbed to Multi-Array® 96-well HighBind microplate wells (Meso Scale Discovery). The microplate wells were blocked with Blocker™ BLOTTO buffer after removing excess capture antibody. Standards and quality control samples, prepared by spiking known quantities of AMG 139 into 100% normal human serum pool, were loaded into the microplate wells after pre-treating with a dilution factor of 100 in Blocker™ BLOTTO buffer, as are samples to be tested and matrix blank. Any AMG 139 in the samples was captured by the immobilized capture antibody. Unbound material was removed by washing the microplate wells. Following washing, SULFO-TAGTM conjugated detection antibody (anti-AMG 139 1A4.1 mAb) was added to the microplate wells to bind captured AMG 139. Unbound SULFO-TAGTM conjugated capture antibody was removed by washing the microplate wells.

Following this washing, Read Buffer T (Meso Scale Discovery) was added to aid in the detection of bound SULFO-TAGTM conjugated detection antibody. When the microplate is electrically stimulated, the SULFO-TAGTM label, in the presence of the co-reactant tripropylamine (TPA) in the read buffer, emits light at 620 nm. The quantity of light emitted is proportional to the amount of AMG 139 bound by the capture antibody in the initial step. Light emission was detected using an appropriate plate reader; for example, a Sector Imager 6000 equipped with Discovery Workbench software. Data were reduced using Watson Laboratory Information Management System data reduction package using a 5PL (autoestimate) (5-parameter logistic) regression model with a weighting factor of 1/Y2. The amount of AMG 139 in a given serum sample was determined by comparison to the standard curve formed by the standards and quality control samples.

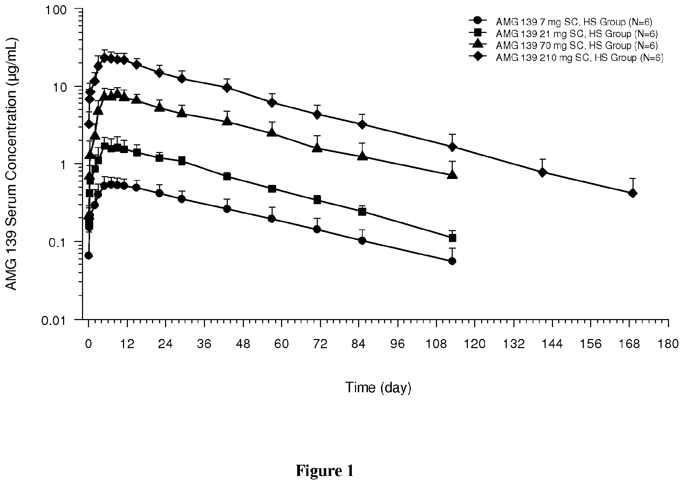

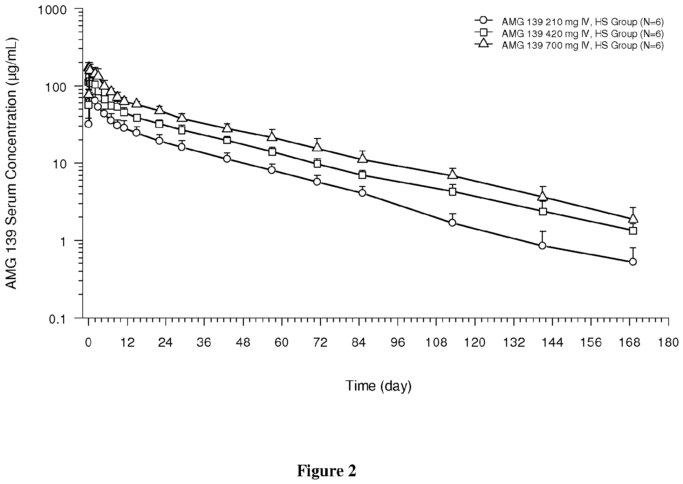

In Part A, the AMG 139 serum concentration versus time profiles for healthy subjects (n = 42) exhibited linear PK, as indicated by serum AMG 139 exposure that increased approximately dose proportionally across all doses tested, except for the 7 mg SC dose (Figures 1 and 2). The median Tₘₐₓ values across doses ranged from 4 to 8 days after a single SC administration (Table 2). Relative bioavailability after a single SC dose was estimated to be 68.9%. Group mean estimates of the terminal half-life after SC or IV administration across all dose levels ranged from 26.6 to 33.0 days, which are typical of an IgG antibody.

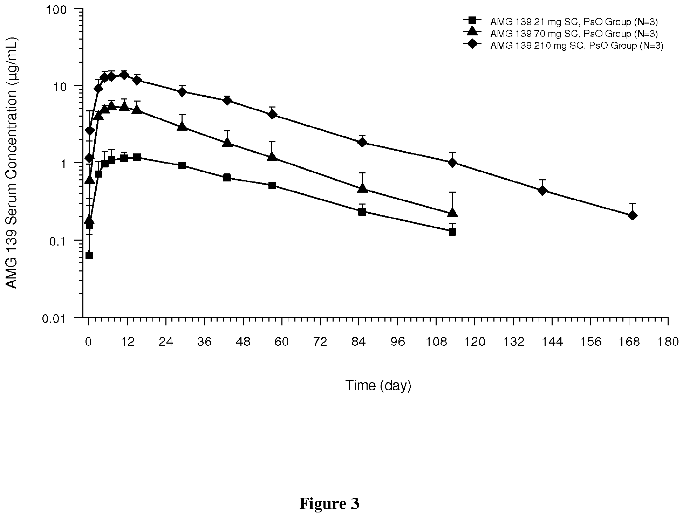

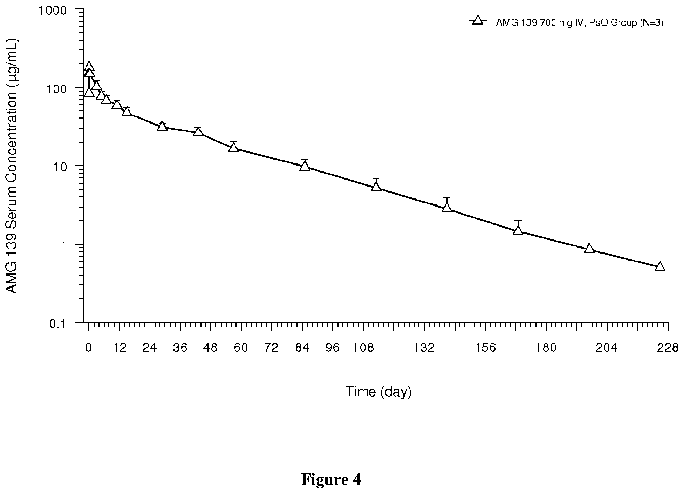

In Part B, the AMG 139 serum concentration versus time profiles for subjects with PsO (n = 12) exhibited linear PK, as indicated by serum AMG 139 exposure that increased approximately dose proportionally across all doses tested in this study (Figures 3 and 4). The median Tₘₐₓ values across doses ranged from 9 to 13 days after a single SC administration (Table 1). Relative bioavailability after a single SC or IV dose was estimated to be 66.9%. Group mean estimates of the terminal half-life after SC or IV administration across all dose levels ranged from 21.6 to 31.0 days, which are typical of an IgG antibody.

In general, AMG 139 PK was similar between healthy subjects in Part A and subjects with PsO in Part B. The one exception was that healthy subjects showed greater exposure (AUC and Cₘₐₓ) of AMG 139 compared to the subjects with PsO. The median Tₘₐₓ after SC administration occurred earlier for healthy subjects than for subjects with PsO. Mean half-life values of AMG 139 were similar between healthy subjects (26.6 to 33.0 days) and subjects with PsO (21.6 to 31.0 days), as were bioavailabilities (68.9% and 66.9%, respectively). Clearance (CL) and volume of distribution (V_{z}) of AMG 139 were consistent across dose levels among healthy subjects (Part A) and among subjects with PsO (Part B).

Patient samples were also tested for binding antibodies to AMG 139. The assay utilized a electrochemiluminescence (ECL) MSD (Meso Scale Discovery) technology platform, which is based on multivalent characteristics of antibody binding. The testing strategy involved a tiered two-assay approach consisting of a screening assay and a specificity assay. Samples with signal to noise ratio (S/N) greater than assay cut point in the screening assay were further tested in the specificity assay by incubating the sample with excess AMG 139 prior to testing.

To enable dissociation of antibody complexes, acid treatment of samples was performed prior to analysis. Acid-treated serum samples and controls were added to a solution consisting of equal parts of biotinylated-AMG 139 (B-AMG 139) and ruthenylated-AMG 139 (Ru-AMG 139) in 1 M Tris, pH 9.5, and are incubated at ambient temperature to allow for anti-AMG 139 antibodies to bind both a B-AMG 139 molecule and a Ru-AMG 139 molecule, thereby forming a complex.

Following the incubation, all samples and controls are transferred to a washed streptavidin-coated standard bind MSD plate blocked with bovine serum albumin and incubated at ambient temperature to allow for the capture of B-AMG 139 and formed complexes on the streptavidin surface. The plate wells are washed and a solution of MSD read buffer containing tripropylamine is added. The plate is read on the MSD Sector Imager 6000 plate reader. Within the instrument, ruthenium participates in an electrochemiluminescent reaction that is triggered when the voltage was applied. The complexes containing the Ru-AMG 139 that are captured on the wells of the plate result in an ECL signal proportionate to the concentration of anti-AMG 139 antibodies in the sample.

None of the 73 subjects in this study developed anti-drug antibodies. Therefore, the potential effects of immunogenicity on AMG 139 disposition could not be assessed.

**Table 2: Mean PK Parameters of AMG 139 After Single-dose SC or IV Administration in Healthy Subjects (Part A) and Subjects with PsO (Part B), Study 20080767**

| **Route** | **Dose (mg)** | **N** | **Cₘₐₓ (µg/mL)** | **tₘₐₓ (day)** | **AUCₗₐₛₜ (day•µg/mL)** | **AUC_{inf} (day•µg/mL)** | **t_{1/2,z} (day)** | **CL^{a} (mL/day)** | **V_{Z}^{a} (L)** |
|---|---|---|---|---|---|---|---|---|---|
| Part A (Healthy Subjects) | | | | | | | | | |
| SC | 7 | 6 | 0.581(20.0) | 6.0(1.0-21) | 26.4(28.2) | 28.9(29.6) | 30.3(13.0) | 256(22.9) | 11.1(22.1) |
| | 21 | 6 | 1.72(33.7) | 8.0(4.0-14) | 71.7(17.3) | 76.2(16.5) | 26.6(11.3) | 282(15.9) | 10.8(21.0) |
| | 70 | 6 | 7.79(23.4) | 8.0(7.8-8.2) | 341(32.9) | 373(35.2) | 29.0(21.0) | 218(51.3) | 8.34(20.5) |
| | 210 | 6 | 24.3(22.2) | 4.0(3.9-10) | 940(28.9) | 1008(27.6) | 27.8(12.3) | 223(30.0) | 8.83(26.3) |
| IV | 210 | 6 | 73.0(20.4) | 0.17(0.17-0.33) | 1430(22.7) | 1471(20.8) | 28.4(11.1) | 147(18.0) | 5.98(15.9) |
| | 420 | 6 | 122(14.9) | 0.17(0.063-0.17) | 2109(24.1) | 2453(13.7) | 33.0(5.6) | 174(13.3) | 8.25(11.3) |
| | 700 | 6 | 175(16.8) | 0.17(0.042-0.33) | 3705(17.6) | 3801(17.8) | 32.9(10.9) | 188(15.0) | 8.93(18.3) |

| Part B (Subjects with PsO) | | | | | | | | | |
|---|---|---|---|---|---|---|---|---|---|
| SC | 21 | 3 | 1.23(18.1) | 13(6.0-13) | 62.2(4.5) | 67.8(4.9) | 29.9(10.6) | 310(4.8) | 13.4(9.8) |
| | 70 | 3 | 5.43(21.6) | 10(6.0-10) | 200(38.6) | 207(40.6) | 21.6(15.6) | 376(38.0) | 11.4(29.1) |
| | 210 | 3 | 13.7(14.5) | 9.0(4.0-9.9) | 630(20.2) | 637(20.3) | 25.6(11.3) | 338(18.2) | 12.4(19.2) |
| IV | 700 | 3 | 157(17.6) | 0.17(0.057-0.33) | 3101(17.6) | 3138(18.0) | 31.0(8.1) | 229(20.1) | 10.2(16.2) |

| | | | | | | | | | |
|---|---|---|---|---|---|---|---|---|---|
| AUC_{inf} = area under the concentration-time curve from time 0 to infinity; AUCₗₐₛₜ = area under the concentration-time curve from time 0 to the time of the last measurable concentration; CL = clearance; Cₘₐₓ = maximum observed concentration post dose; %CV = coefficient of variation; F = bioavailability; IV = intravenous(ly); PsO = psoriasis; SC = subcutaneous(ly); t_{1/2,z} = elimination half-life; tₘₐₓ = time to maximum observed concentration; V_{z} = volume of distribution; V_{z}/F = apparent volume of distribution PK parameters are reported as mean (CV%) with 3 significant figures except for tₘₐₓ which is reported as median(min-max) rounded to 2 significant figures. %CV is reported to 1 decimal place. ^{a} CL and V_{z} represent CL/F and V_{z}/F for SC administration. | | | | | | | | | |

### Example 2

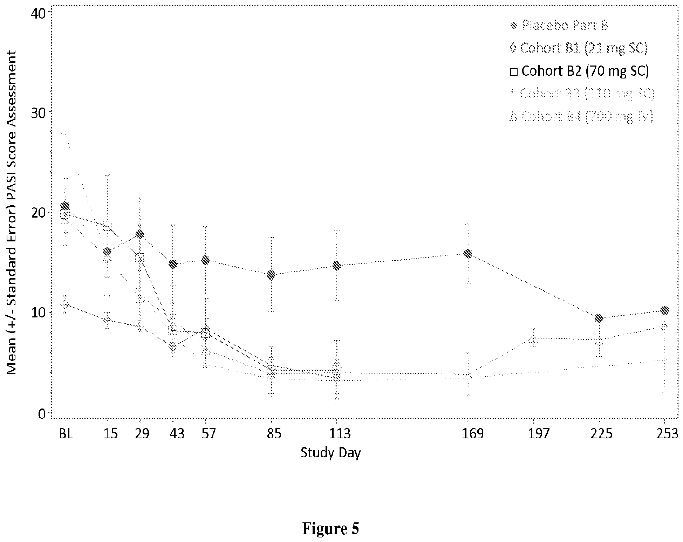

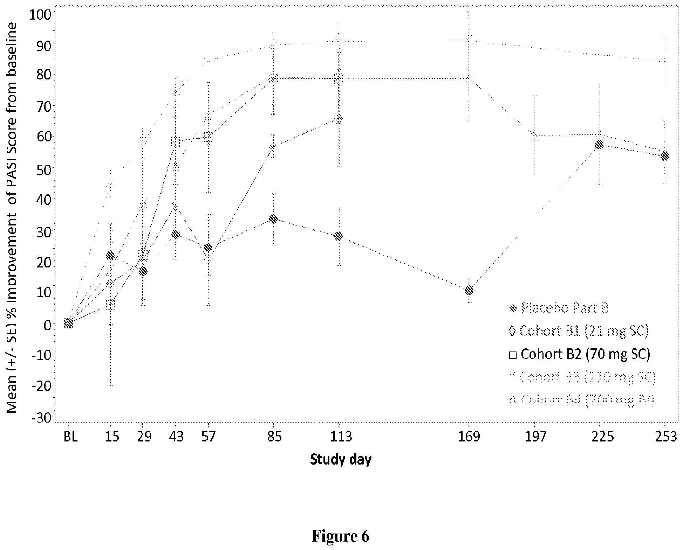

The efficacy of AMG 139 was evaluated in subjects with PsO as a secondary endpoint in the previously mentioned Phase 1a FIH study (20080767, Part B). Reductions in mean Psoriasis Area and Severity Index (PASI) scores (Figures 5 and 6, and Table 3), mean target lesion scores, and mean Physician Global Assessments (PGAs) occurred in all AMG 139 treatment groups compared with the placebo group. Even though the numbers of subjects per treatment group were small, it was clearly apparent that the single administrations of AMG 139 were efficacious with respect to degree and duration of responses in treatment groups receiving doses as low as 70 mg SC. Efficacy was also apparent from the number and percent of subjects reaching PASI 50, PASI 75, or PASI 90 over time by treatment group, and from the number and percent of subjects reaching PASI 50, PASI 75, PASI 90, or PASI 100 at any time during the study by treatment group. For any given dose, treatment effects (PASI, target lesion score, PGA) appeared to reach their maximum by approximately Day 85 to Day 113. Mean percent changes from baseline in PASI score was as high as approximately 90% (i.e., at Days 85, 113, and 169 in the AMG 139 210 mg SC group). Results past Day 113 for the AMG 139 210 mg SC and 700 mg IV groups suggested that AMG 139 treatment effects from the single doses began to return toward baseline after approximately Day 169. With group-mean terminal half-life values for AMG 139 mostly being in the range of 25 to 30 days, circulating levels of AMG 139 by Day 169 were approximately 1% to 2% of Cₘₐₓ for any given dose. Photographs of subjects and subject lesions were also taken at various time points. Overall, these photographs were visually and qualitatively consistent with the PASI, target lesion assessment, and PGA results.

**Table 3: Summary of Subjects Reaching PASI 50, PASI 75, PASI 90, or PASI 100 at Any Time During the Study (Part B: Study 20080767)**

| **PASI Response** | **Treatment Group** | | | | |
|---|---|---|---|---|---|
| | **Placebo Part B N = 5 n (%)** | **Cohort B1 21 mg SC N = 3 n (%)** | **Cohort B2 70 mg SC N = 3 n (%)** | **Cohort B3 210 mg SC N = 3 n (%)** | **Cohort B4 700 mg IV N=3 n (%)** |
| PASI 50 | 2 (40) | 3 (100) | 3 (100) | 3 (100) | 3 (100) |
| PASI 75 | 0 (0) | 1 (33) | 3 (100) | 3 (100) | 2 (67) |
| PASI 90 | 0 (0) | 1 (33) | 1 (33) | 2 (67) | 1 (33) |
| PASI 100 | 0 (0) | 0 (0) | 0 (0) | 2 (67) | 0 (0) |

| | | | | | |
|---|---|---|---|---|---|
| IV = intravenous; N = number of subjects with PASI score at baseline; PASI = Psoriasis Activity and Severity Index; SC = subcutaneous | | | | | |

### Example 3

A quantitative population pharmacokinetics (pop PK) model for AMG 139 was established to simulate the PK of future dosing regimens, as well as incorporation with a quantitative PK/pharmacodynamic model for simulating AMG 139 efficacy. The pop PK model was based on healthy subject and PsO patient data described above.

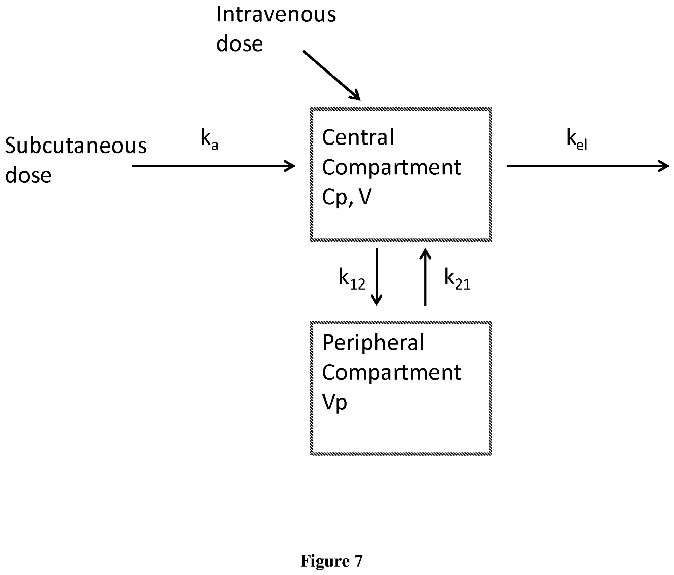

Pop PK modeling of subcutaneous (SC; 7, 21, 70, or 210 mg) or intravenous (IV; 210, 420, or 700 mg) doses was performed with NONMEM v7.2. Data analysis used individual PK data fit simultaneously to a structural two-compartment model with first-order elimination from the central compartment and first-order absorption from a depot compartment (Figure 7). The inter-subject variability parameters and residual error model were varied to obtain the lowest objective function. Body weight and disease were explored as potential PK covariates.

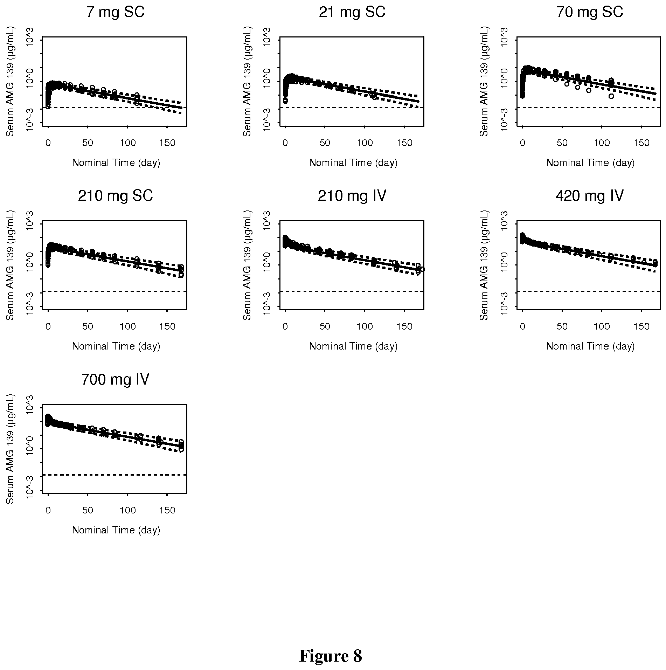

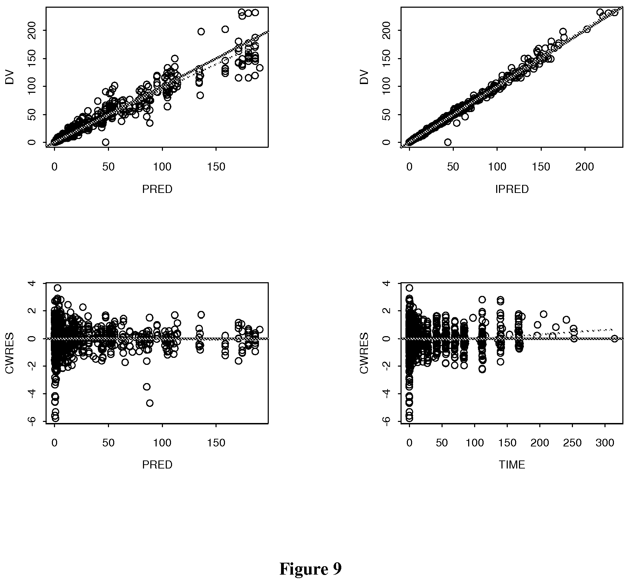

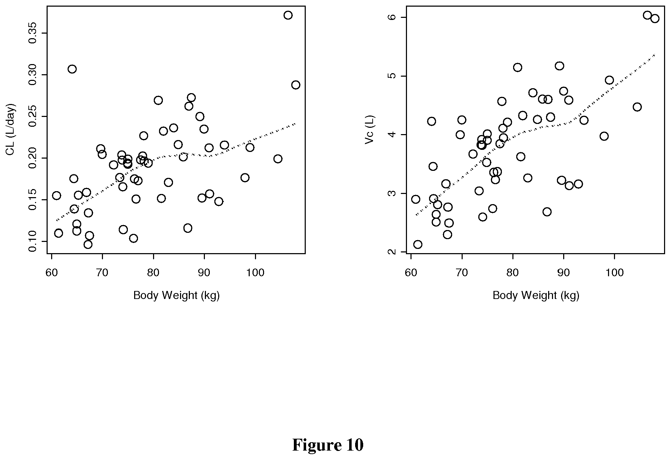

The final AMG 139 pop PK model predicted mean concentration-time profiles that fit the data well within 90% confidence intervals (Figure 8), and visual predictive diagnostic plots show strong correlations between observed and predicted values (Figures 8 and 9). Absorption rate constant, systemic clearance (CL), and central volume of distribution (V_{c}) were 0.242 h⁻¹, 0.171 L/day, and 3.58 L, respectively, with inter-individual variability of 66%, 24%, and 20%, respectively (Table 4). Body weight as a covariate had power coefficient values of 1.04 and 1.11 for CL and V_{c}, respectively, and showed a positive correlation with CL and V_{c} (Figure 10). After adjusting for body weight, the additional effect of a disease status covariate on CL [1.13-fold increase (0.93-1.3, 95% CI)] did not show a statistically significant improvement on the model in this Phase 1 study dataset.

**Table 4: Population PK Model Parameter Estimates after Single Dose Administration of AMG 139 to Healthy Volunteers and Psoriasis Subjects**

| Parameter | Parameter estimate | SE | Inter-individual variability (%) | SE |
|---|---|---|---|---|
| ka (hr⁻¹) | 0.242 | 0.0354 | 66 | 9 |
| CL (L/day) | 0.171 | 0.0149 | 24 | 3 |
| V_{c} (L) | 3.58 | 0.318 | 20 | 2 |
| Vₚ (L) | 3.16 | 0.322 | 25 | 3 |
| Q (L) | 0.576 | 0.107 | 90 | 15 |

The AMG 139 pop PK model established utility for simulating AMG 139 PK in future inflammatory disease populations, as well as incorporation with ongoing efficacy studies for establishment of a PK/pharmacodynamic model.

These results support several dosing regimens for administering an anti-IL-23 antibody to an individual afflicted with a psoriatic condition that is associated with the IL-23 pathway. An appropriate dosing regimen can be selected from the dosing regimens shown in Table 5 below.

**Table 5: Dosing Regimens**

| |
|---|
| 21 mg SC or IV every 1 month (0.5 - 1.5 months); 21 mg includes amounts in the range of 15 - 54 mg |
| 70 mg SC or IV every 3 months (1.5 - 4.5 months); 70 mg includes amounts in the range of 55 - 149 mg |
| 210 mg SC or IV every 6 months (4 - 8 months); 210 mg includes amounts in the range of 150 - 299 mg |
| 700 mg SC or IV every 6 months (4 - 12 months); 700 mg includes amounts in the range of 300 - 1100 mg |

### Example 4

Immunophenotyping was a primary endpoint in Part A and B of this study. Lymphocyte populations including T cells, B cells, NK cells, regulatory T cells (Tregs), and Th17 cells were quantified over time by flow cytometry performed on whole blood samples.

Whole blood was collected from subjects at indicated time points in potassium EDTA (ethylenediaminetetraacetic acid)-containing glass tubes and processed within 24 hours (Cohorts A1-A7) or following a 24 hour incubation period at room temperature (Cohorts B1-B4). The CYTO-STAT tetraCHROME® staining kit (Beckman Coulter, Fullerton, CA) was used for enumeration of T (CD3+, CD4+ and CD8+), B and NK cell populations. Other T cell populations (Treg and Th17 cells) were identified in separate tubes using custom combinations of fluorochrome-conjugated monoclonal antibodies.

After antibody staining of whole blood, red blood cells were lysed in all samples using Coulter IMMUNOPREP reagent system (Beckman Coulter) and fixed in a 1% solution of paraformaldehyde. For blood stained with markers for T, B, and NK cells, samples were then immediately analyzed by flow cytometry. Blood stained with markers for Treg and Th17 cell populations was washed prior to analysis by flow cytometry.

### Flow cytometry

Data acquisition and analysis was performed using a FC500 flow cytometer (Beckman Coulter) with a single blue laser (488 nm) and a 5-color optical configuration. An initial lymphocyte gate was set on the CD45-expressing population exhibiting low side scatter characteristics. T cell (including Treg and Th17) and NK T cell populations were derived from the CD3+ subset of lymphocytes. NK and B cell populations were identified from the non-CD3 expressing subset of lymphoctyes.

The CYTO-STAT tetraCHROME® staining kit (Beckman Coulter) allows for enumeration of T, B, NK and NK T cells using Flow-Count Fluorospheres (Beckman Coulter) using a single platfrom; therefore, these populations are reported as absolute counts. Treg and Th17 cells are reported as percentages of total CD3+ CD4+ cells since Flow-Count Fluorospheres were not included in these tests. Absolute counts for these populations can be calculated using the clinical lymphocyte counts from each enrolled subject on the day of immunophenotypic analysis.

No directional changes in quantities of the above-mentioned types of lymphocytes or in frequencies of CD4+ or CD8+ lymphocytes expressing the above-mentioned cytokines upon in vitro stimulation were observed in AMG 139-treated subjects. Fewer differentially regulated genes (lesional/nonlesional) were observed after treatment of PsO subjects with AMG 139, consistent with a drug-related PD effect.

In view of the above, it will be appreciated that the invention also encompasses the following items:
1. A method of treating psoriasis in a subject in need thereof comprising administering to the subject an anti-IL-23 antibody in an amount and at an interval of:
   a. 15 - 54 mg every 0.5 - 1.5 months;
   b. 55 - 149 mg every 1.5 - 4.5 months;
   c. 150 - 299 mg every 4 - 8 months; or
   d. 300 - 1100 mg every 4 - 12 months.
2. The method of item 1, wherein the amount and interval are:
   a. 15 - 21 mg every 0.5 - 1.0 month;
   b. 55 - 70 mg every 1.5 - 3.0months;
   c. 150 - 260 mg every 4 - 6 months; or
   d. 300 - 700 mg every 4 - 8 months.
3. The method of item 1, wherein the amount and interval are:
   a. 21 mg every month;
   b. 70 mg every 3 months;
   c. 210mg every 6 months; or
   d. 700 mg every 6 months.
4. The method of item 1, wherein the amount and interval are:
   a. 210 mg every 3 months or
   b. 700 mg every 3 months.
5. The method of item 1, wherein the amount and interval are:
   a. 210 mg every 1 month or
   b. 700 mg every 1 month.
6. A method of treating psoriasis in a subject in need thereof comprising administering to the subject an amount of an anti-IL-23 antibody in an amount and at an interval sufficient to achieve and/or maintain a quantity of anti-IL-23 antibody per volume of serum of between 12.5 ng/ml and 1000 ng/ml.
7. The method of item 6, wherein the quantity of an anti-IL-23 antibody per volume of serum is at least 10 ng/ml.
8. The method of item 6, wherein the quantity of an anti-IL-23 antibody per volume of serum is selected from the group consisting of: at least 25 ng/ml; at least 50 ng/ml; at least 60 ng/ml; at least 70 ng/ml; at least 75 ng/ml; and at least 80 ng/ml.
9. The method of item 6, wherein the quantity of an anti-IL-23 antibody per volume of serum is between 85 ng/ml and 100 ng/ml.
10. The method of item 6, wherein the quantity of an anti-IL-23 antibody per volume of serum is between 70 ng/ml and 150 ng/ml.
11. The method of item 6, wherein the quantity of an anti-IL-23 antibody per volume of serum is is between 50 ng/ml and 250 ng/ml.
12. The method of item 6, wherein the quantity of an anti-IL-23 antibody per volume of serum is is between 40 ng/ml and 500 ng/ml.
13. The method of item 6, wherein the quantity of an anti-IL-23 antibody per volume of serum is between 25 ng/ml and 750 ng/ml.
14. The method of item 6, wherein the quantity of an anti-IL-23 antibody per volume of serum is between 10 ng/ml and 1,000 ng/ml.
15. The method according to any of the above items , wherein the anti-IL23 antibody is administered IV.
16. The method according to any of the above items, wherein the anti-IL23 antibody is administered SC.
17. The method according to any of the above items, wherein the anti-IL-23 antibody is AMG 139.

## Claims

1. An anti-IL-23 antibody for use in treating psoriasis, wherein the anti-IL-23 antibody is administered in an amount and at an interval of:
a. 15 - 54 mg every 0.5 - 1.5 months;
b. 55 - 149 mg every 1.5 - 4.5 months;
c. 150 - 299 mg every 4 - 8 months; or
d. 300 - 1100 mg every 4 - 12 months.

2. The anti-IL-23 antibody for the use according to claim 1, wherein the amount and interval are:
a. 15 - 21 mg every 0.5 - 1.0 month;
b. 55 - 70 mg every 1.5 - 3.0 months;
c. 150 - 260 mg every 4 - 6 months; or
d. 300 - 700 mg every 4 - 8 months.

3. The anti-IL-23 antibody for the use according to claim 1 or claim 2, wherein the amount and interval are:
a. 21 mg every month;
b. 70 mg every 3 months;
c. 210mg every 6 months; or
d. 700 mg every 6 months.

4. The anti-IL-23 antibody for the use according to claim 1 or claim 2, wherein the amount and interval are:
a. 210 mg every 3 months;
b. 700 mg every 3 months;
c. 210 mg every 1 month; or
d. 700 mg every 1 month.

5. An anti-IL-23 antibody for use in treating psoriasis, wherein the anti-IL-23 antibody is administered in an amount and at an interval sufficient to achieve and/or maintain a quantity of anti-IL-23 antibody per volume of serum of
a. between 12.5 ng/ml and 1000 ng/ml; or
b. at least 10 ng/ml.

6. The anti-IL-23 antibody for the use according to claim 5, wherein the quantity of an anti-IL-23 antibody per volume of serum is selected from the group consisting of: at least 25 ng/ml; at least 50 ng/ml; at least 60 ng/ml; at least 70 ng/ml; at least 75 ng/ml; and at least 80 ng/ml.

7. The anti-IL-23 antibody for the use according to claim 5, wherein the quantity of an anti-IL-23 antibody per volume of serum is between 85 ng/ml and 100 ng/ml.

8. The anti-IL-23 antibody for the use according to claim 5, wherein the quantity of an anti-IL-23 antibody per volume of serum is between 70 ng/ml and 150 ng/ml.

9. The anti-IL-23 antibody for the use according to claim 5, wherein the quantity of an anti-IL-23 antibody per volume of serum is between 50 ng/ml and 250 ng/ml.

10. The anti-IL-23 antibody for the use according to claim 5, wherein the quantity of an anti-IL-23 antibody per volume of serum is between 40 ng/ml and 500 ng/ml.

11. The anti-IL-23 antibody for the use according to claim 5, wherein the quantity of an anti-IL-23 antibody per volume of serum is between 25 ng/ml and 750 ng/ml.

12. The anti-IL-23 antibody for the use according to claim 5b, wherein the quantity of an anti-IL-23 antibody per volume of serum is between 10 ng/ml and 1,000 ng/ml.

13. The anti-IL-23 antibody for the use according to any of the above claims, wherein the anti-IL23 antibody is administered IV.

14. The anti-IL-23 antibody for the use according to any of the above claims, wherein the anti-IL23 antibody is administered SC.

15. The anti-IL-23 antibody for the use according to any of the above claims, wherein the anti-IL-23 antibody comprises the heavy and light chain variable regions of SEQ ID NO:1 and SEQ ID NO:2, respectively.
